# FASCICULE DE BREVET EUROPEEN

(11) **EP 0 912 599 B1**
(45) Date de publication et mention de la délivrance du brevet: **28.11.2001**
(21) Numéro de dépôt: 97930606.5
(22) Date de dépôt: 27.06.1997
(51) Int. Cl.: C07J 51/00, A61K 31/575

(54) **COMPLEXES ORGANO-METALLIQUES A BASE DE STEROLS ET DE DIGLYCERIDES ET COMPOSITIONS PHARMACEUTIQUES ET PRODUITS DIETETIQUES EN CONTENANT**
ORGANOMETALLISCHE KOMPLEXE VON STERIN VERBINDUNGEN UND GLYKOSIDEN UND PHARMAZEUTISCHE ZUSAMMENSETZUNGEN UND DIÄTETISCHE PRODUKTE DAVON
DIGLYCERID AND STEROL BASED ORGANOMETALLIC COMPLEXES AND PHARMACEUTICAL COMPOSITIONS AND DIETETIC PRODUCTS CONTAINING THEM

(30) Priorité: 03.07.1996 FR 9608263
(43) Date de publication de la demande: 06.05.1999
(73) Titulaire: Maurel Santé, 34160 Castries-l'Amaury (FR)
(72) Inventeur: MAUREL, Jean-Claude, F-78490 Montfort L'Amaury (FR); CHAPUIS, Jean-Marc, F-92800 Puteaux (FR); MONGOLD, Jean-Jacques, F-34990 Juvignac (FR); JOUY, Nicolas, F-34090 Montpellier (FR)
(74) Mandataire: Giraud, Françoise
(86) Numéro de dépôt international: FR9701153
(87) Numéro de publication internationale: WO9801461

(56) Documents cités:
- WO-A-79/00435
- WO-A-96/23811
- FR-A- 2 686 511
- FR-A- 2 686 512
- FR-A- 2 686 514
- FR-A- 2 686 515
- US-A- 5 244 887
- CHEMICAL ABSTRACTS, vol. 110, no. 9, 27 février 1989 Columbus, Ohio, US; abstract no. 069237, M. IVORRA ET AL: "Antihyperglycemic and Insulin-Releasing Effects of beta-Sitosterol 3-beta-D-glucoside and its Aglycone, beta-Sitosterol" colonne 1; XP002002377 & ARCH. INT. PHARMACODYN. THER., vol. 296, 1988, pages 224-231,
- CHEMICAL ABSTRACTS, vol. 116, no. 19, 11 mai 1992 Columbus, Ohio, US; abstract no. 187365, G. CROS ET AL: "Effects of Vanadyl Derivatives on Animal Models of Diabetes" page 2; colonne 2; XP002002379 & MOL. CELL. BIOCHEM., vol. 109, no. 2, 1992, pages 163-166,
- CHEMICAL ABSTRACTS, vol. 121, no. 1, 4 juillet 1994 Columbus, Ohio, US; abstract no. 580, M. NAKAI ET AL: "Insulin-Like Effects of Selenium Compounds and the Mechanism" page 64; colonne 1; XP002002380 & BIOMED. RES. TRACE. ELEM., vol. 4, no. 2, 1993, pages 81-82,
- CHEMICAL ABSTRACTS, vol. 116, no. 5, 3 février 1992 Columbus, Ohio, US; abstract no. 34320, J. MCNEILL ET AL: "Insulinlike Effects of Sodium Selenate in Streptozocin-Induced Diabetic Rats" page 64; colonne 1; XP002002381 & DIABETES, vol. 40, no. 12, 1991, pages 1675-1678,
- CHEMICAL ABSTRACTS, vol. 79, no. 23, 10 décembre 1973 Columbus, Ohio, US; abstract no. 133416, I. DINU ET AL: "Influence of Chromium on the Activity of Some Hepatic Enzymes" page 70; colonne 2; XP002002382 & REV. ROUM. BIOCHIM., vol. 10, no. 2, 1973, BUCHAREST, RO, pages 105-111,
- CHEMICAL ABSTRACTS, vol. 77, no. 11, 11 septembre 1972 Columbus, Ohio, US; abstract no. 70845, G. BERENSHTEIN ET AL: "Interaction of Selenium and Vitamin E on Indexes of Carbohydrate Metabolism in Animals" page 103; colonne 2; XP002002383 & VESTSI AKAD. NAVUK BELARUS. SSR, SER. BIYAL. NAVUK, no. 2, 1972, pages 53-57,
- CHEMICAL ABSTRACTS, vol. 61, no. 5, 31 août 1964 Columbus, Ohio, US; abstract no. 5179a, V. VASIL'EVA ET AL: "Molecular Compounds of beta-Sitosterol with Metal Chlorides" colonne 5179; XP002002384 & ZH. OBSHCH. KHIM., vol. 34, no. 5, 1964, pages 1400-1401,
- CHEMICAL ABSTRACTS, vol. 103, no. 1, 8 juillet 1985 Columbus, Ohio, US; abstract no. 442, J. W. MALAISSE ET AL: "Stimulation of Protein Kinase C and Insulin Release by 1-Oleoyl-2-acetyl-glycerol" page 44; colonne 1; XP002020682 & EUR. J. BIOCHEM., vol. 149, no. 1, 1985, pages 23-27,
- CHEMICAL ABSTRACTS, vol. 93, no. 5, 4 août 1980 Columbus, Ohio, US; abstract no. 46986, S. S. GERAS'KINA ET AL: "Synthesis of Provitamin D3 from Plant Raw Material" page 969; colonne 1; XP002020683 & KHIM.-FARM. ZH., vol. 13, no. 12, 1979, pages 67-70,

## Description

La présente invention concerne à titre de produits industriels nouveaux des complexes organométalliques à base de stérols et de diglycérides.

Elle concerne aussi des compositions pharmaceutiques et des produits diététiques contenant ces complexes.

Les acylglycérols, plus particulièrement les acylglycérols d'acides gras, sont présents dans la plupart des végétaux et sont des constituants majoritaires des corps gras végétaux et animaux.

Seuls varient d'un acylglycérol à l'autre le nombre d'acides gras, leur position sur le glycérol, leur longueur de chaine et le nombre et la position de leurs éventuelles insaturations. Il peut s'agir en particulier, de mono-, di-, ou triacylglycérols.

Le sitostérol et le sitostanol, dont il existe pour chacun deux isomères actifs le β et le γ sont également des constituants de l'ensemble des végétaux ; dans le texte, le terme sitostérol comprend le béta ou le gamma sitostérol (ou un mélange de ces deux isomères), et le terme sitostanol comprend le béta ou le gamma sitostanol (ou un mélange de ces deux isomères).

Certains extraits totaux de plantes qui, comme la majorité des plantes, contiennent parmi leur très nombreux constituants des flavonoïdes, tanins, saponines, coumarines, alcaloïdes, triterpènes, stérols, carbohydrates et/ou glycosides ont été décrits pour leur activité hypoglycémiante, comme par exemple l'extrait d'accacia (Egypt. J. Pharm. Sci., 1992, 33 (1-2), 327-340), ou l'extrait de teucrium oliveranium (Fitoterapia, 1984, 55(4), 227-230). Aucun lien entre l'activité et la fraction contenant les stérols n'est établi. Dans ces publications, cette activité est en général attribuée aux carbohydrates ou polyols du cyclohexane comme le condutirol, dérivé de l'inositol isolé à partir d'un extrait de gymnéma sylvestre qui ne diminue pas directement la glycémie, mais empèche les sucres de passer à travers la paroi intestinale.

Quelques sapogénines ont été décrites pour le traitement du diabète, (par exemple la sarsasapogénine et la similagénine dans EP 0 204 661). Il s'agit de dérivés dont la chaine latérale est constituée de deux éthers cycliques spiro.

D'autres dérivés hypoglycémiant s issus de végétaux ont été décrits comme par exemple:
- le glycoside de la leucopélargonidine dans Indian J. Exp. Biol. 1993, 31(1), 26-29 ;
- les glycosides de la prototimosaponine dans Chemical Abstracts 117(9):83451f qui résume le brevet japonais JP 04054194 ;
- le ginsenoside Rb2 décrit dans Chemical Abstracts 105(17):146237r qui résume le brevet japonais JP 61024597. Ce produit testé sur le rat agit comme antidiabétique, la dose, par voie intrapéritonéale, étant de 10 mg/par rat. Cette dose diminue la glycémie de 20%.

Les feuilles et les sommités fleuries de l'ortie (urtica dioica) sont traditionnellement connues pour leurs propriétés antidiabétiques ( première description dans la littérature scientifique dès 1926). Cette activité est reproductible et quantifiable, mais personne n'en a jusqu'à présent isolé le ou les principes actifs.

Les stérols végétaux, en particulier ceux de la racine d'ortie (urtica dioica) font depuis quelques années l'objet de beaucoup d'études notamment pour le traitement de certaines tumeurs comme l'adénome prostatique.

Un extrait végétal de pygeum africanum, commercialisé en France sous le nom de TADENAN® par les Laboratoires DEBAT pour le traitement de l'adénome prostatique depuis de nombreuses années, a fait l'objet d'études récentes tendant à prouver que l'activité serait dûe à la fraction stérol-lipidique qui contient d'après R. Longo et S. Tyra, Farmaco, Ed. Pract. 1983, 38(7), pages 287-292, du béta-sitostérol, le béta-sitostéryl glucoside et la 3 béta-sitosténone ainsi que du n-docosanol et du n-docosyl trans-férulate, d'après le résumé Chemical Abstracts CA 104(22):193255j.

Dans le Journal of Natural Products, 1987, 50(5), pages 881-885, N. Chaurasia et M. Wichtl décrivent l'isolation, la séparation, et l'identification de certains stérols de la racine de l'ortie.

En 1979, une équipe de chercheurs brésiliens décrivait dans Rev. Med. Univ. Fed. Ceara, 19(1-2), 49-53, un extrait de grains verts de café arabica dont le sitostérol serait hypoglycémiant. Depuis lors, cette activité n'a pu être reproduite et aucun travail publié ne l'a confirmée.

Le Brevet US 4 588 717 décrit comme complément vitaminique, réducteur de poids et complément de régime réduisant l'absorption des carboydrates, et donc la glycémie, un mélange contenant des métaux, des complexes de métaux avec du fructose, des vitamines, des acides gras, des phospholipides, des esters de stérols , des amino-acides, etc.

Les auteurs de ce brevet ne décrivent pas à proprement parler d'activité thérapeutique, ils n'attribuent pas les propriétés de leur mélange à une seule famille de produits présents dans le mélange.

Les vertus hypocholestérolémiantes des stérols des végétaux sont connus depuis plusieurs décennies. Ils réduisent la cholestérolémie en inhibant l'absorption intestinale du cholestérol (par co-précipitation ou par déplacement des liaisons des micelles). L'adjonction à l'alimentation de quantités importantes de ces stérols, en particulier le sitostérol à la dose journalière de 30 g minimum est utilisé depuis les années cinquante pour traiter l'hypercholestérolémie, mais le mauvais goût et la texture déplaisante de ces préparations ont été un obstacle à son utilisation. Une telle activité ne peut être obtenue qu'avec des quantités importantes de sitostérol.

Miettinen et coll.( New England Journal of Medicine, 1995, vol. 333, n°20, p.1308) ont développé une margarine contenant l'ester du sitostanol. Le sitostanol réduit davantage l'absorption intestinale du cholestérol selon ces auteurs. Une étude en double insu a été menée pendant une année dans une population modérément hypercholestérolémique; 51 sujets ont consommé une margarine enrichie en ester de sitostanol ( 2,6 g/j).Au bout d'un an, on a observé chez ces patients une réduction de 10% de la cholestérolémie totale, et une réduction des LDL de 14%. Aucune modification des taux de triglycérides ni du HDL n'ont été observé. Il faut noter cependant que l'étude a été menée dans une population ayant un apport élévé en cholestérol, et les auteurs doutent que l'on puisse avoir une activité comparable chez des sujets ayant un régime pauvre en cholestérol, car le sitostanol n'est pas absorbé et son activité se limite à l'inhibition de l'absorption du cholestérol au niveau intestinal.

Une activité hypocholestérolémiante a également été décrite chez l'animal : Antihypercholesterolemic activity in rabbits : I. Ikeda et all., J. Nutr. Sci. Vitaminol., 27 , 243, 1981. Ils ajoutent à l'alimentation des lapins du cholestérol et du sitostérol ou du sitostanol (à la dose de 0,5% et O,2%). Le sitostanol inhibe la montée du taux plasmatique du cholestérol mieux que le sitostérol. Les auteurs concluent que l'activité hypocholestérolémiante du sitostanol résulte de effet inhibiteur de l'absorption du cholestérol au niveau intestinal.

Aucun traitement n'existe actuellement pour corriger l'hyperinsulinisme et ses conséquences métaboliques, à l'exception du régime alimentaire de type méditérannéen et une activité physique musculaire régulière.

Par ailleurs, le diabète est controlé ou contenu par des produits qui régulent la glycémie:
- insuline exogène;
- sulfamides et/ou biguanides;
- produits réduisant l'absorption des carbohydrates;
- inhibiteurs de l'aldose réductase non encore commercialisés.

Aucun de ces traitements ne traite réellement les causes du diabète, ni ne provoque de rémission vraiment durable, ni de guérison qui persiste après l'arrêt du traitement.

Le traitement des dyslipidémies repose actuellement sur deux types de traitement:
- les fibrates: ils réduisent modérément le taux de cholestérol, et de façon plus significative l'hypertriglycéridémie;
- les statines ( inhibiteurs de la HMG CoA réductase): elles réduisent l'hypercholestérolémie et n'ont aucune action sur les triglycérides.
Ces deux types de traitement de l'hypercholestérolémie et de l'hypertriglycéridémie sont de plus en plus utilisée car l'on estime aujourd'ui qu'une baisse de 10% du taux de cholestérol entraine une diminution de 20% du risque de maladie coronarienne; ils sont trés fréquement mal tolérés par les patients chez qui ils entrainent des atteintes musculaires douloureuses.

Différents brevets ont décrit differentes associations, en particulier sous forme de complexes organo-métalliques utilisables dans le traitement et/ou la prévention de l'hyperglycémie, du diabète et des maladies associées. On citera:
- les brevets français FR 2 654 620 et FR 2 676 738 qui décrivent des dérivés organiques de métaux de transition, à structure porphirinique;
- le brevet français FR 2 659 333 qui décrit des dérivés à structure phospholipidique;
- le brevet français FR 2 686 600 qui décrit des complexes organo-métalliques de l'inositol ou de ses dérivés;
- le brevet français FR 2 686 511 qui décrit des complexes d'acides aminés;
- le brevet français FR 2 686 512 qui décrit des complexes organo-métalliques avec des dérivés catécholiques;
- le brevet français FR 2 686 514 qui décrit des complexes organo-métalliques dans lesquels le ligand contient un cycle renfermant au moins deux atomes d'azote;
- le brevet français FR 2 686 603 qui décrit des complexes organo-métalliques avec des dérivés de l'acide dithiocarbamique;
- le brevet français FR 2 686 515, plus particulièrement orienté vers l'utilisation des dérivés organo-métalliques du niobium pour le traitement et la prévention des désordres du métabolisme des glucides et/ou des lipides.

Par ailleurs, la demande de brevet français FR 2 695 390 décrit des compositions pharmaceutiques contenant des complexes de tanins avec différents métaux.

Poursuivant ses recherches approfondies en vue de déterminer des fractions végétales actives ou des complexes de ces fractions avec des métaux, la demanderesse a découvert, tout à fait par hasard, que certains produits, fractions ou complexes par ailleurs inactifs ou insuffisamment actifs comme agent hypoglycémiant voyaient leur activité considérablement augmentée lorsqu'ils étaient administrés en solution dans l'huile d'olive. Ceci a conduit la demanderesse à chercher parmi les nombreux constituants de l'huile d'olive ceux qui pouvaient conduire par mélange ou réaction avec les produits testés à des produits plus actifs.

C'est ainsi que la demanderesse a pu identifier de nouveaux produits constitués de complexes organo-métalliques et obtenu par réaction entre un dérivé du vanadium au degré d'oxydation 4 ou 5 et deux composés organiques issus d'extraits végétaux et constitués respectivement de sitostérol, et d'acylglycérols.

Ces produits sont décrits dans la demande internationale WO-A- 96/23811 qui décrit des complexes organo-métalliques susceptibles d'être obtenus par réaction:
- d'un cation d'un métal (M) au degré d'oxydation au moins égal à 2 utilisable comme biocatalyseur dans le métabolisme vivant,
- du béta ou du gamma sitostérol libre ou d'un mélange de ces deux produits ou d'un extrait végétal en contenant,
- d'un mono-, d'un di-, ou d'un triglycéride répondant à la formule (I): 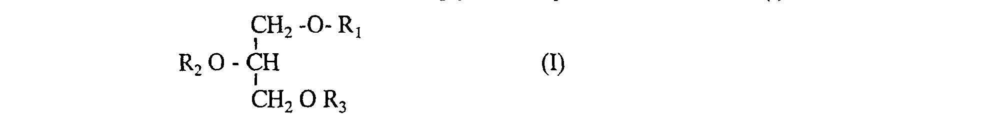
dans laquelle:
- R₁ est un reste acyle d'acide gras en C₁₄ à C₂₄ saturé ou non, de l'hydrogène, ou un mono-, un di-, ou un tri-galactose ou glucose,
- R₂ est un reste acyle d'acide gras en C₁₈ présentant une insaturation, de préférence un reste d'acide oleique ou l'un de ses isomères de position de la double liaison ( cis-6,7,9,11 et 13) ou l'un de ses isomères isoramifiés,
- R₃ est un reste acyle d'acide gras en C₁₄ à C ₂₄ saturé ou non ou un atome d'hydrogène.

Les produits décrits dans ce document corrigent durablement des animaux diabétiques, et ce , quel que soit le modèle de diabète animal, aussi bien d'origine génétique que chimique. Ces mêmes produits corrigent l'hyperinsulinisme et l'insulino-resistance d'un modèle animal similaire à l'hypertension artérielle dite « essentielle » chez l'homme avec une correction simultanée de l'hyperinsulinisme et de l'hypertension artérielle.

Des complexes analogues peuvent être préparés à partir d'autres dérivés métalliques de métaux au degré d'oxydation au moins égal à 2 et connus pour leur activité antidiabétique.

Des complexes analogues sont également susceptibles de se former entre les deux types de dérivés organiques cités précédemment et différents cations de métaux connus comme cations à activité biocatalytique, ces complexes constituant, dans tout les cas, des agents particulièrement efficaces comme transporteur de cations à activité biocatalytique.

Par cations à activité biocatalytique, on entend aussi bien ceux ayant une activité biocatalytique directe, que ceux susceptibles de se substituer à des biocatalyseurs et modifier par ce moyen certaines voies métaboliques pathologiques. On citera l'exemple du vanadium qui peut se substituer au phosphate du fait de leur similitude de chimie de coordination: ainsi les phosphatases acides et alcalines sont inhibées par les vanadyles et les vanadates, de même que la tyrosine phosphatase qui induit une stimulation de la phosphorylation de la tyrosine du récepteur périphérique de l'insuline ainsi que des proteines kinases associées.

La demanderesse, poursuivant ses recherches dans le même domaine, a maintenant identifié une nouvelle famille de complexes organo-métalliques à base de diglycérides bien déterminés qui présente une activité particulièrement importante dans le même domaine.

Elle a également découvert que le sitostérol commercial extrait du soja contenait environ 15 à 20% de sitostanol; ainsi le sitostérol, le sitostanol, ou un mélange de ces deux composés (par exemple le sitostérol commercial) possèdent cette même activité lorsqu'ils entrent dans la composition des complexes selon l'invention.

Ainsi selon un premier aspect, l'invention concerne selon une de ses caractéristiques essentielles de nouveaux complexes organo-métalliques susceptibles d'être obtenus par réaction:
- d'un cation d'un métal (M) au degré d'oxydation au moins égal à 2 utilisable comme biocatalyseur dans le métabolisme vivant,
- du sitostérol, du sitostanol ou d'un mélange de sitostérol et de sitostanol, ou d'un extrait végétal contenant du sitostérol, du sitostanol ou un mélange de sitostérol et de sitostanol,
- du diglycéride répondant à la formule (I) : 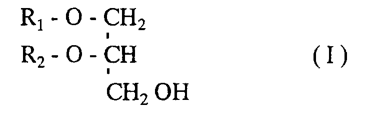
dans laquelle :
- R₁ est un reste acyle d'acide oleique (C_{18 :1}),
- R2 est un reste acyle d'acide gras comprenant entre 2 et 18 atomes de carbone, linéaire ou ramifié, saturé ou insaturé.

Selon une variante préférée, R₂ est un groupement acétyle ou oleoyle, de préférence acétyle.

Les cations du métal M utilisables pour préparer les complexes selon l'invention sont tous les cations de degré d'oxydation au moins égal à 2 et susceptibles de se complexer avec les deux types de dérivés organiques ci-dessus et connus pour leur activité catalytique sur les systèmes biologiques. On parlera dans le présent mémoire de biocatalyseur pour désigner ces métaux à activité catalytique sur les systèmes biologiques.

A titre d'exemples, si l'on vise un produit à activité hypolipémiante, ou hypoglycémiante et/ou antidiabétique et/ou insulinomimétique, on choisira tout particulièrement un dérivé métallique du vanadium, du niobium, du molybdène, du sélénium, du chrome ou du zinc.

Dans ces dérivés métalliques :
- le vanadium est avantageusement au degré d'oxydation 3 , 4 ou 5, de préférence 4,
- le niobium est avantageusement au degré d'oxydation 4 ou 5, de préférence 5,
- le sélénium est avantageusement au degré d'oxydation 4 ou 6, de préférence 4,
- le molybdène est en général à un degré d'oxydation compris entre 3 et 6, de préférence 3,
- le chrome est préférentiellement au degré d'oxydation 3,
- le zinc est de préférence au degré d'oxydation 2.

A titre d'exemples de métaux adaptés à d'autres types d'application, on citera
- l'antimoine ou l'étain, si l'on vise le traitement des maladies auto-immunes touchant en particulier le système nerveux, par exemple la sclérose en plaques ( SEP),
- l'or dans les pathologies auto-immunes touchant l'appareil locomoteur, par exemple dans le cas de la polyarthrite rhumatoïde,
- le vanadium dans les néoplasies du tube digestif, en particulier du pancréas, du colon et du rectum,
- le ruthénium ou le palladium dans les néoplasies respiratoires,
- le lithium dans les pathologies du système nerveux central,
- l'étain dans les syndromes d'immuno-défisciences acquises.

A titre d'exemples de dérivés métalliques particulièrement utile selon l'invention, on citera les halogénures, les oxyhalogénures, les sulfates les hydrates, les sels d'ammonium, les méthalates de métaux alcalins ou alcalino-terreux qui peuvent être avantageusement dissous dans l'eau ou parfois dans des alcools. On citera des dérivés organiques de métaux comme les acétylacétonates, les alcoolates ou des complexes de métaux avec des solvants organiques, par exemple des éthers, le THF, la DMF. Ces dérivés organiques de métaux sont généralement solubles dans des solvants organiques, plus particulièrement dans des solvants chlorés comme le chloroforme ou le dichlorométhane.

Le métal pourra aussi être apporté par une extraction préalable dans des aliments ou végétaux connus pour leur riche teneur en ce métal (poivre noir, persil, basilic, aneth...).

Le sitostérol sera utilisé de préférence en mélange avec le sitostanol, avec une quantité relative d'au moins 15% de sitostanol.Le sitostérol et le sitostanol pourront être utilisés purs.

Le sitostérol existe sous forme commerciale, mais il est alors en mélange avec du campestérol et du sitostanol . Dans ces produits commerciaux généralement extraits du soja, le sitostérol représent 50 à 70 % du produit qui est mélangé avec du campestérol et du sitostanol dans des quantités respectives de l'ordre de 15% chacun.

Dans un premier temps, l'activité paraissait exclusivement liée à la présence du sitostérol puisqu'il existait une relation effet-dose entre l'activité et la quantité de sitostérol; de plus le campestérol pur ne montrait aucune activité. Poursuivant ses travaux, la demanderesse a découvert que la quantité de sitostanol présente dans le mélange variait dans la même proportion que le sitostérol ; enfin le sitostanol pur présente une activité identique à celle du mélange sitostérol / sitostanol tel que défini précedemment.

Le sitostanol ou stigmastanol pur est un produit commercial qui est obtenu par hydrogénation catalytique du sitostérol. Sa synthèse à partir du béta-sitostérol a été 1937, Bernstein, Wallis, J. Org. Chem Soc., 2, 341.

On peut également préparer le sitostérol, et donc le sitostanol par extraction à partir de végétaux selon des techniques révélées dans la littérature, par exemple à la page 95 de la thèse soutenue à Montpellier par Claude Cerdon ayant pour titre « Modulation de la production des sapogénines stéroïdiques en réponse à l'inhibition de la synthèse des stérols ».

Cette extraction est avantageusement réalisée par complexation des métaux selon le procédé décrit en particulier dans le brevet français n° 2 316 247 dans lequel est décrit un procédé pour isoler les 3-hydroxystéroïdes et les 3-oxostéroïdes de mélange contenant ces composés.

Pour réaliser cette extraction, on pourra utiliser tout végétal ou produit d'origine végétale connu pour sa teneur relative élévée en sitostérol.

A titre d'exemples de végétaux ou de produits d'origine végétale à teneur relative élevée en sitostérol libre, on citera en particulier l'huile d'olive, l'huile de soja, les feuilles de coton, les feuilles de café, les germes de blé, dont les teneurs relatives en stérols libres ainsi que le pourcentage de sitostérol dans la fraction stérol libre sont donnés dans le tableau ci-dessous :

| **ESPECES** | **teneur / kg** | **% de la fraction stérol** |
|---|---|---|
| Huile d'olive | 1 310 mg | 91 % |
| Huile de soja | 1 908 mg | 53 % |
| Feuilles de coton | 3 961 mg | 93 % |
| Feuilles de café | 9 914 mg | 51 % |
| Germe de blé | 17 336 mg | 67 % |

Les teneurs relative en sitostanol n'ont pas été étudiées.

Il faut également préciser que la fraction stérol libre contient une proportion variable selon les végétaux des isomères 24 R et 24 S, proportion mal connue car peu ou pas étudiée.
Cette proportion, de même que la quantité relative de sitostanol que l'on ne peut séparer du sitostérol lors des purifications, pourraient expliquer la meilleure activité relative de la fraction stérol de certains végétaux, et surtout l'excès de sitostérol nécessaire à la fabrication des nouveaux produits décrits dans l'invention.

Un certains nombre d'entre eux et tout particulièrement ceux qui s'avèrent les plus actifs dans les applications visées existent également sous forme commerciale. C'est le cas en particulier du 1-oleoyl-2-acetyl glycérol, du 1, 2-dioleoyl glycérol qui existent sous forme commerciale avec une pureté d'environ 98%. Le 1-oleoyl-2-acetyl glycérol est, en particulier obtenu par synthèse chimique ou biochimique, en particulier par acetylation en 2 par l'acetyl CoA du 1, 2-dioleoyl glycérol, ou encore à partir d'un dioleylphospholipide, en particulier la phosphatidylcholine qui est présente dans l'huile d'olive, et qui est cassée par la phospholipase C en une dioleine et une phosphorylcholine.

Par ailleurs les acylglycérols utiles pour la préparation des complexes selon l'invention peuvent être isolés dans la plupart des végétaux.

Ces acylglycérols peuvent être extraits de labiées, orties (urtica dioïca et urens), la sauge, les bugles, la luzerne ou alfafa (medicago sativa), les eucalyptus (globulus, delegatensis), angelica archangelica et angelica sinensis, les ombellifères, le gymnema sylvestre (asclepiadaceae), le marsdenia condurango, le momordica charantia, le gingko biloba, le chardon marie, le thé vert, le thé noir (camelia sinensis), la rhubarbe, le dioscorea dumetorum (dioscoreaceae), l'indigofera arrecta (papilionaceae), le pittosporaceae), l'agrimonia eupatoria, le curcuma xanthorrhiza (roxb.), l'uncaria gambier (roxb.), le swertia chirayita (roxb.), les résédacées (reseda phyteuma, lutea, alba, luteola), l'harpagophytum, les rubiacées, les gentianaceaes, l'asparagus racemosus, l'aubépine (crataegus oxyacantha), le gui (viscum album), les paletuviers (rhizophoracées), le kaki, les chènes, le chène à galle (fagacées), les ronces, l'hamamelis (hamamelidacées), le ratanhia (krameriacées), la salicaire (lithracées), le calophyllum (clusiacées), les acacias, l'acacia à cachou (légumineuses mimosoidées), le quebracho (anacardiacées), les raisins (vitis vinifera, ampelidacées), les cassis (saxifragacées dont ribes nigrum), les myrtilles (ericacées), les mures (rubus fructicosus), le sureau, le choux rouge, l'ail (allium sativum), le coriandre (coriandrum sativum), le genévrier (juniperus communis), les pins (abietacées), le pin maritime, le cyprés (cupressus sempervirens), les hibiscus, les rhus (anacardiacées), les dicotylédones, les fougères, les gymnospermes, les mélianthus, les rosacées, les roses, le malva verticillata (malvaceae), les fraisiers, les citrus (rutacées), la benoite, les châtaigniers (fragacées), la bistorte, les légumineuses, le sophora, le lespedeza, le sophora, les polygonacées, le sarrazin.

On utilise de façon particulièrement avantageuse comme source d'acylglycérols des huiles végétales insaturées , en particulier de l'huile d'olive de première pression à froid.

D'une façon générale, on choisira comme source d'acylglycérols utiles se lon l'invention une huile contenant une forte teneur en acide oleique, une telle huile contenant généralement une forte proportion d'acylglycérols utiles selon l'invention.

A titre d'exemples de telles huiles, on citera :
- l'huile d'olive dont la teneur en acide oleique (C_{18:1}) est comprise entre 60 et 80%, les huiles européennes étant plus riches en C_{18:1} que les huiles d'Afrique du Nord,
- l'huile de tournesol de variété dite hybride tournesol oleique, contenant 83% de C_{18:1} au lieu de 16% dans l'huile de tournesol normale,
- l'huile de carthame de variété oleique, contenant de 73 à 80% de C _{18:1} au lieu de 10 à 20% dans la variété linoleique,
- l'huile d'amande contenant de 64 à 82% de C _{18:1},
- l'huile de noisette contenant de 66 à 83% de C _{18:1},
- l'huile d'avocat dont la teneur en C _{18:1} varie entre 36 et 80%.

La fraction contenant des acylglycérols utiles pour préparer les complexes selon l'invention peut être avantageusement préparée à partir de l'huile d'olive de la façon suivante: on effectue une prépurification de l'huile d'olive en la passant sur une faible hauteur de silice ( 10 à 15 cm), en réalisant le vide, et en éluant à l'aide d'un solvant organique tel que le dichlorométhane ou un mélange de cyclohexane et d'acetate d'ethyle dans des proportions 96/4 ou tout autre éluant présentant une polarité voisine, de façon à isoler les triglycérides présents dans l'huile. La silice est ensuite lavée avec de l'acétate d'ethyle afin de récupérer les diglycérides interessants selon l'invention ainsi que la fraction monoglycéride et des composés minoritaires polaires (dont la phosphatidylcholine).

La fraction ainsi obtenue est ensuite passée sur une colonne de silice et éluée avec différents gradients de mélange acétate d'ethyle / cyclohexane compris entre 10/90 et 100/0 de façon à séparer les différentes familles chimiques de l'huile et à récupérer la famille active recherchée.

Le 1,2-dioleoyl glycérol est obtenu après la sortie sur la colonne des diglycérides 1-3.

Le 1-oleoyl-2-acetyl glycerol est o btenu juste avant le 1-oleoyl glycerol et le 2-oleoyl glycérol.

Les complexes selon l'invention sont, comme on l'a vu précedemment, aisément fabriqués par simple mélange des trois types de composés décrits ci-dessus. Ce mélange est avantageusement réalisé dans un solvant organique tel que le dichlorométhane, l'ether, l'acétate d'éthyle, l'éthanol.

Le complexe peut également être réalisé directement en utilisant comme solvant une huile , qui peut être ensuite utilisée comme solvant d'injection pour le produit actif dans le cas d'une forme injectable, ou comme excipient pour une forme orale.

Le mélange est maintenu pendant quelques heures à une température comprise entre 30° et 40°C ; on peut également obtenir le même résultat avec quelques expositions flash à une température de l'ordre de 50° à 60°C pendant quelques minutes.

En ce qui concerne les proportions des différents constituants, elles peuvent varier dans une assez large gamme. Toutefois, en corrélant l'activité des produits obtenus avec les proportions des trois types de réactifs mis en jeu, on a pu déterminer les proportions suivantes, données en se référant à une mole de métal :
- le sitostérol et/ou le sitostanol, plus généralement le mélange sitostérol/sitostanol ou le sitostanol pur sont avantageusement utilisés dans des proportions de 1 000 à 10 000 en mole par rapport au métal,
- le diglycéride est avantageusement utilisé dans des proportions équimolaires par rapport au métal. Toutefois, ces proportions peuvent être telles que le rapport en mole diglycéride/métal soit compris entre 1 et 100.

Ce rapport varie par ailleurs, avec la nature du diglycéride.

En particulier, le rapport diglycéride/métal sera avantageusement compris entre 1 et 100 dans le cas où le diglycéride est le 1-2-dioleoyl glycérol.

On choisira avantageusement un rapport compris entre 1 et 10 dans le cas où le diglycéride est le 1-oleoyl-2acétyl glycérol.

A titre d'exemple , pour 50 mg d'un mélange de sitostérol/sitostanol ( dans des proportions 80/20 telles qu'obtenues après purification du soja), et pour une quantité de vanadium exprimée en métal de 1 à 10 *µ*g, on utilisera de préférence de 1 à 2 mg de 1-2 dioleoyl glycérol ou 20 à 500 *µ*g d'oleoyl acetyl glycérol.

Les différents constituants sont formellement identifiés par des moyens analytiques adaptés :
- pour le sitostérol et le sitostanol :chromatographie en phase gazeuse, HPLC, RMN¹H,
- pour l'acylglycérol : HPLC avec un détecteur à diffusion de lumière, sur une colonne kromasil C₁₈, en présence d'un éluant constitué par exemple d'acétonitrile isocratique.

Le pic de masse du complexe n'est pas détectable avec les méthodes usuelles, telles que l'ionisation chimique et l'impact électronique, ce qui peut s'expliquer par le fait que les complexes formés par ces deux constituants avec le métal sont généralement instables, comme la plupart des complexes organo-métalliques ayant une activité biologique.

Il est apparu que le complexe obtenu était actif avec des doses beaucoup plus basses d'acylglycérols, lorsqu'en position 2 du glycérol était une chaine acylée plus courte, entre 2 et 18 carbones, et particulièrement lorsqu'en 2 se trouvait un acétyle.

Ainsi, on obtient la même activité en synthétisant le complexe organo-métallique avec 10 à 100 fois moins d'oleoylacétyl glycérol que de dioleoyl glycérol.

Par ailleurs, tant pour le 1-2-dioleoyl glycérol que pour le 1-oleoyl-2-acétyl glycérol,
c'est l'isomère naturel le « sn » qui possède la meilleure activité.

Le métal biocatalytique M est avantageusement choisi parmi 1 es métaux suivants : zinc, fer, cuivre, magnésium, vanadium, titane, chrome, manganèse, cobalt, nickel, gallium, germanium, antimoine, étain, indium, palladium, rhodium, ruthénium, technium, molybdène, niobium, zirconium, yttrium, tantale, tungstène, rhénium, osmium, iridium, platine, or, argent, thallium.

D'une façon particulièrement avantageuse, le métal est le vanadium, au degré d'oxydation 3,4 ou 5, de préférence 4 ou 5, de préférence encore 4. Ceci permet une application des complexes selon l'invention, en particulier comme hypolipémiant, et dans le traitement et/ou la prévention du diabète et de ses complications.

Selon un autre de ses aspects, l'invention concerne l'utilisation des complexes décrits précédemment en tant qu'agents transporteurs de cations à activité biocatalytique, les ligands liés au métal permettant d'accroître la biodisponibilité du dit métal.

Une telle application est d'autant plus importante que, d'une façon générale, l'homme du métier sait que la difficulté d'utilisation thérapeutique des cations métalliques est liée à leur toxicité aux doses actives : des exemples bien connus sont les sels de lithium utilisés en pathologie psychiatrique, ou les sels de platine, ruthénium, palladium utilisé en cancérologie.

L'exemple du vanadium dans le traitement du diabète et de ses complications illustre bien ce problème et la solution apportée par la présente invention. En effet :
- les sels de vanadium en développement depuis une décennie (vanadyl et vanadate) sont actifs chez l'animal à la dose de 5 à 10 mg/kg (exprimé en métal) par voie intrapéritonéale; à cette dose, ils sont très toxiques, et ne peuvent être administrés à l'homme.
- certains complexes organiques décrits au cours des dernières années, notamment dans le brevet européen n° O 305 264 déposé le 10/08/88, permettent d'obtenir une activité à des doses de l'ordre de 1 à 5 mg/kg (exprimé en métal).
- les complexes organométalliques de vanadium , décrits selon l'invention, permettent quant à eux, une activité optimale (retour à une glycémie normale et guérison le plus souvent durable de l'ensemble des symptomes) avec une dose de vanadium (exprimée en métal) de l'ordre de 1 *µ*g/kg chez l'animal, donc à des doses nettement plus basses (5 000 fois moins) et dénuées de toxicité, comparable aux doses alimentaires quotidiennes ( 40 à 60 *µ*g par jour chez l'homme).

Les complexes organo-métalliques décrits dans la présente invention optimisent ainsi la biodisponibilité du cation métallique transporté, permettant son utilisation thérapeutique avec une toxicité faible ou nulle, ce qui représente un avantage considérable par rapport à l'état de la technique.

Selon un autre de ses aspects, l'invention concerne également des compositions pharmaceutiques contenant au moins un complexe tel que définis précédemment et un véhicule, excipient ou support pharmaceutiquement acceptable.

Selon une première variante, la composition pharmaceutique contient un complexe tel que défini précédemment d'un seul métal.

Selon une autre variante, la composition phar maceutique peut comprendre un mélange de deux complexes tels que définis précédemment de deux métaux différents.

Un tel mélange aboutit, dans certains cas, à un effet synergique : c'est le cas en particulier, lorsque la composition contient un mélange de complexes de vanadium et de zinc. On a pu observer, dans ce cas, un effet synergique lors de l'utilisation de composition pharmaceutique contenant un mélange de ces deux types de complexes dans le traitement du diabète sur certains modèles animaux.

Comme excipient, véhicule ou support pharmaceutiquement acceptable, on peut utiliser tout excipient, véhicule ou support bien connu de l'homme de l'art. On peut citer par exemple, et de façon non limitative : le lactose, l'amidon de maïs, le glucose, le saccharose, les agents sucrants tels que le sirop de maltitol, la gomme arabique, la gélatine, les carrhagénanes, l'acide stéarique ou le stéarate de magnésium, la dextrine, les maltodextrines, le mannitol, le talc, les corps gras d'origine naturelle, en particulier les huiles d'origine végétale riches en acides gras insaturés et en stérols. En particulier, si cela s'avère éventuellement nécessaire, on peut utiliser d'autres additifs bien connus de l'homme de l'art tels que des stabilisants, des desséchants, des liants, des tampons de pH.

Les compositions de l'invention peuvent être administrées de différentes manières, en particulier par voie orale, avec une absorption buccale ou digestive, par voie per-muqueuse, sous-cutanée, intra-musculaire, transdermique (sous forme de patch ou de gel).

Selon un autre de ses aspects, l'invention concerne l'utilisation des complexes de l'invention pour la préparation d'un médicament destiné à être utilisé comme agent régulateur ou stimulant des systèmes biocatalytiques, en particulier dans le traitement ou la prévention des déficits ou des dysfonctionnements ( génétiques ou acquis) des systèmes enzymatiques nécéssitant la présence de métaux sous forme de cations comme catalyseur des réactions biochimiques , notamment dans le traitement ou la prévention des maladies métaboliques, auto-immunes ou néoplasiques.

Dans toutes ces utilisations, on met en application le fait que la biodisponibilité du métal lui-même connu comme biocatalyseur se trouve accrue du fait de sa complexation.

La complexation conduit dans tout les cas à une exaltation du pouvoir biocatalytique du métal, ce qui permet d'obtenir une activité avec des doses de métal considérablement diminuées par rapport aux doses habituellement utilisées.

Selon une des variantes particulièrement importantes de l'invention, on citera l'utilisation des complexes dans lequel le métal est choisi dans le groupe constitué du vanadium, du niobium, du sélénium, du chrome et du molybdène pour le traitement et/ou la prévention des complications cardio-vasculaires liées à l'hypercholestérolémie et/ou l'hypertriglycéridémie, au diabète insulino-dépendant ou non insulino-dépendant, et à l'insulino-résistance; ces pathologies sont en particulier l'hypertension artérielle, les coronaropathies obstructives (infarctus du myocarde, angor) , les microangiopathies oculaires ou périphériques; il s'agit aussi de l'obésité de type androïde qui accompagne l'insulino-resistance.

On choisira parmi ces complexes de préférence ceux du vanadium au degré d'oxydation 3,4 ou 5, de préférence encore les complexes dans lesquels le vanadium se trouve au degré d'oxydation 4.

De façon avantageuse pour la préparation de produits diététiques contenant le complexe selon l'invention, le vanadium pourra être apporté en effectuant son extraction avec un solvant d'origine naturelle tel qu'une huile végétale ou de l'eau déminéralisée; on citera comme aliments riches en vanadium le poivre noir, le persil, le basilic, l'aneth , certains champignons...; selon une autre méthode, le vanadium pourra être apporté direcetement en utilisant le produit alimentaire pulvérisé, par exemple du poivre noir pulvérisé qui apporte
340 *µ*g de vanadium par gramme de poivre.

Les exemples suivants sont donnés à titre purement illustratif de l'inv ention.

### EXEMPLES

### Exemple 1 : Préparation d'un complexe selon l'invention

### a) Préparation d'une fraction contenant plus de 90% d'oleoyl acétyl glycérol

On part d'huile d'olive que l'on prépurifie sur une colonne de silice de faible hauteur (10 à 15 cm). On réalise l'élution avec le dichlorométhane ou un mélange cyclohexane/acétate d'éthyle 96/4, sous vide. Cette opération permet d'éliminer les triglycérides présents dans l'huile .

La silice est ensuite lavée avec de l'acétate d'éthyle et on récupère la fraction interessante de l'huile.

Cette fraction est ensuite passée sur colonne de silice en éluant avec différents gradients de mélange acétate d'éthyle/cyclohexane compris entre 10/90 et 100/0. Cette opération permet de séparer les différentes familles chimiques de l'huile et de récupérer la fraction active recherchée qui est controlée en HPLC avec détecteur à diffusion de lumière.

L'oleoyl acetyl glycérol est récupéré en tête de la fraction monoglycéride.

### b) Préparation du complexe selon l'invention

On dissout dans 15 cc de dichlorométhane :
- 1 g d'un mélange commercial de sitostérol / sitostanol dans des proportions 70/15 , le reste étant constitué d'impuretés, en particulier le campestérol.
- 1,1 mg de VO(Acac)²,
- 5 mg d'oleoyl acétyl glycérol obtenu par purification de l'huile d'olive.

On chauffe le mélange avec une agitation pendant une heure.

On évapore le solvant et on récupère le complexe.

### c) autre exemple de préparation d'un complexe selon l'invention

On dissout dans 15 cc de dichl orométhane :
- 1 g de sitostanol commercial
- 1,1 mg de VO(Acac)²,
- 5 mg d'oleoyl acétyl glycérol obtenu par purification de l'huile d'olive.

On chauffe le mélange à plusieurs reprises à 50°C pendant quelques minutes.

On évapore le solvant et on récupère le complexe.

### Exemple 2 - Préparation d'un complexe selon l'invention en utilisant un glycéride commercial

On dissout dans 15 cc de dichlorométhane :
- 1 g de sitostanol commercial
- 1,1 mg de VO(Acac)²,
- 20 mg de 1-2-dioleoyl glycérol commerci al.

On chauffe le mélange à plusieurs reprises à 50°C pendant quelques minutes.

On évapore le solvant et on récupère le complexe.

### Exemple 3 : Préparation d'une composition pharmaceutique

On prépare une composition pharmaceutique à partir de l'un des complexes formés selon l'exemple 1 ou 2, de la façon suivante :

Le complexe est incorporé au liquide utilisé comme excipient ou comme solvant d'injection, et le solvant est chassé par exemple sur un évaporateur rotatif. Le produit peut alors être administré.

Le complexe peut également être incorporé à un patch pour une diffusion transdermique.

Le complexe peut aussi sous sa forme solide être conditionné en gélules gastro-resistantes.

### Exemple 4 : Préparation d'un complexe selon l'invention

On dissout dans 15 cc de dichlorométhane :
- 1 g de sitostanol commercial
- 100 *µ*g de VO(Acac)²,
- 10 mg de 1-oleoyl-2- acétyl-sn- glycérol commercial, que l'on maintient à une température inférieure à 0°C jusqu'au moment d'effectuer le mélange des composants pour éviter qu'il évolue vers une position 1-3 qui n'a plus d'activité.

On ajoute 20 cc d'huile de soja codex ; on maintient le mélange en l'agitant pendant 30 minutes à 40°C, puis on évapore le solvant ; le produit actif est prêt pour être injecté.

### Exemple 5 : Préparation d'une composition pharmaceutique selon l'invention

On dissout dans 150 ml d'huile d'olive de première pression à froid (qui contient les acylglycérols nécessaires à la réalisation du complexe selon l'invention),
- 15 g de sitostanol commercial;
- 1,5 mg de VO(Acac)²;

On maintient le mélange en agitation pendant une heure à 35°C. Aucun solvant organique autre que l'huile n'est utilisé ; le produit est prêt à être absorbé per os.

### Exemple 6 : Préparation d'un produit diététique ayant un usage médical

- 150 ml d'huile d'olive de première pression à froid;
- 1 g de poivre noir pulvérisé (340 *µ*g de vanadium );
- 15 g de sitostanol commercial;

On maintient le mélange en agitation pendant une heure à 35°C. Aucun solvant organique autre que l'huile n'est utilisé . Le complexe selon l'invention est formé, et le produit est prêt à être absorbé per os .

### Exemple 7 : tests pharmacologiques

Le complexe obtenu selon l'exemple 3 , à partir de la préparation I b , est évalué selon la procédure suivante :
- des rats mâles de souche Wistar provenant de l'élevage de la Société Iffa-Credo et pesant en moyenne 160 g sont maintenus 4 jours en observation et reçoivent de la nourriture et de l'eau de boisson ad libitum. Ils sont soumis à une température de 21°C ± 1°C, et à un cycle jour/nuit de 12 heures.
- ils sont ensuite anesthésiés à l'éther éthylique et on leur administre une dose de 60 mg/kg de streptozotocine en solution dans un tampon citrate à pH 4,5 par une injection
   pratiquée au niveau de la veine du pénis (modèle de rats dit STZ);
- trois jours plus tard, les animaux ( qui pèsent alors 200 g environ) présentant une glycémie comprise entre 3 et 4,9 g/l sont groupés par lot de 6 animaux, 3 par cage et soumis au traitement par la substance à tester par injection intra-péritonéale (désignée ci-après par IP) en solution dans une fraction extraite d'huile d'olive et ne contenant que des triglycérides qui ont un comportement neutre par rapport aux complexes à tester.

On constitue également un lot de rats témoins diabétiques, qui reçoivent le même volume de triglycérides extraits de l'huile d'olive, par voie IP.

Le contrôle de la glycémie est effectué au temps souhaité avec un Glucometter III AMES (Bayer) sur glucofilm, lorsqu'il s'agit d'un test d'évaluation d'un effet hypoglycémiant à 2 et 6 h après l'administration, ou avec un Glucometter I AMES, sur glucostix lorsqu'il s'agit d'un traitement de plusieurs jours, servant à évaluer le rôle régulateur de la glycémie et un éventuel effet rémanent après l'arrêt du traitement; le prélèvement d'une goutte de sang lors de chaque dosage est effectué par incision de l'extrémité de la queue.

Le tableau I ci-dessous donne les résultats obtenus lors du traitement de différents lots de rats en suivant le protocole du paragraphe précédent, et en utilisant le produit préparé selon l'exemple 3, avec le complexe décrit dans l'exemple I b. Le tableau I ci-dessous donne, pour 3 lots de 6 rats diabétiques STZ, les résultats obtenus aux jours JO, J4, J8, et J11 du traitement.

Ce tableau précise aux jours J4, J8, J11:
- le taux de glycémie,
- la variation de ce taux de glycémie par rapport au taux à JO
- le nombre de rats corrigés ; par rats corrigés on entend ceux présentant une glycémie infèrieure à 1,50 g/l,
- la variation de poids par rapport à JO (poids).

| **JO** | **J4** | | | | **J8** | | | | **J11** | | | |
|---|---|---|---|---|---|---|---|---|---|---|---|---|
| glycémie | glycémie | variation | corrigés | poids | glycémie | variation | corrigés | poids | glycémie | variation | corrigés | poids |
| 3,17 | 2,12 | 32% | 2 | 2 | 1,43 | 55% | 5 | 11 | 2,05 | 35% | 4 | 25 |
| 3,23 | 1,85 | 43% | 4 | -2 | 1,77 | 45% | 4 | 14 | 1,70 | 47% | 4 | 23 |
| 3,48 | 2,31 | 34% | 2 | -5 | 1,69 | 51% | 4 | 14 | 1,89 | 46% | 4 | 28 |

Le tableau II ci-dessous donne les résultats obtenus pour 1 lot de 6 rats diabétiques STZ, en suivant que le même protocole que précédemment, en utilisant le produit tel que décrit dans l'exemple 3, en utilisant le complexe préparé dans l'exemple 1 c.

| **JO** | **J4** | | | | **J8** | | | | **J11** | | | |
|---|---|---|---|---|---|---|---|---|---|---|---|---|
| glycémie | glycémie | variation | corrigés | poids | glycémie | variation | corrigés | poids | glycémie | variation | corrigés | poids |
| 3,73 | 2,47 | 34% | 1 | 19 | 2,42 | 35% | 2 | 37 | 2,33 | 38% | 4 | 54 |

### Exemple 8 : Test clinique

Une étude clinique a été menée sur 10 malades présentant une dyslipidémie, 7 hommes et 3 femmes. L'age moyen était de 61 ans.
Le traitement a été instauré pendant une durée de 30 jours; un bilan lipidique a été réalisé avant le traitement et à la fin du traitement.

Le produit donné est celui décrit à l'exemple 5 à la dose de 5 ml par jour, correspondant à une dose de vanadium exprimé en métal de 10 *µ*g par jour.

Aucune modification du régime alimentaire n'a été proposée.

Les résultats sont décrits dans le tableau 3 ci-dessous :

| | | | **CHOLESTEROL** | | **CHOLESTEROL TOTAL /HDL** | | **TRIGLYCERIDES** | |
|---|---|---|---|---|---|---|---|---|
| **VALEUR NORMALE** | | | **inférieur à 2,40 g/l** | | **inférieur à 4** | | **0,50 à 1,50 g/l** | |
| | NOM | AGE | avant traitement | après traitement | avant traitement | après traitement | avant traitement | après traitement |
| 1 | LE G. | 46 | 2,67 | 2,47 | 5,28 | 3,32 | 2,64 | 0,92 |
| 2 | REN. | 74 | 2,94 | 2,30 | 5,15 | 4,34 | 2,10 | 1,50 |
| 3 | NOU. | 66 | 3,51 | 2,43 | 3,69 | 2,47 | 1,07 | 0,65 |
| 4 | CAR. | 69 | 2,91 | 2,77 | 4,16 | 4,26 | 2,11 | 1,75 |
| 5 | LET. | 74 | 2,71 | 2,85 | NR | NR | 1,89 | 2,65 |
| 6 | DUF. | 48 | 1,97 | 2,28 | 6,15 | 5,56 | 1,57 | 2,01 |
| 7 | MAR. | 62 | 3,04 | 2,18 | NR | NR | 2,19 | 1,33 |
| 8 | KOH. | 48 | 2,90 | 1,98 | 6,30 | NR | 0,94 | 0,73 |
| 9 | CAR. | 64 | 3,25 | 3,35 | NR | NR | 0,81 | 0,51 |
| 10 | ALB. | 64 | 3,20 | 3,00 | 4,10 | 3,70 | 0,69 | 0,63 |
| **MOYENNE** | | **61** | **2,91** | **2,56** | **4,98** | **3,94** | **1,60** | **1,27** |

### Analyse des résultats :

| | Différence avant/ après traitement | | |
|---|---|---|---|
| | Cholestérol | Cholestérol / HDL | Triglycérides |
| Moyenne | - 0,35 | -0,81 | -0,33 |
| Variance | 0,24 | 0,51 | 0,46 |

Les calculs sur les moyennes montrent une amélioration sur les trois variables étudiées ( cholestérol, cholestérol total / HDL) et triglycérides).
Une différence significative est mise en évidence pour :
- la variable cholestérol total/HDL au risque d'erreur α de 5% avec un degré de signification inférieure à 0,02.
- la variable cholestérol au risque d'erreur α de 5% avec un degré de signification inférieur à 0,03.
- la variable triglycéride quoique bien améliorée, n'est pas significative avec ce nombre ce cas ; le nombre de cas estimé nécessaire pour un résultat significatif est de 13 patients !

L'activité hypolipémiante mise en évidence, n'est pas en relation exclusive avec :
- le cholestérol : en effet par comparaison avec la publication décrivant l'activité hypocholestérolémiante du cholestérol chez l'homme (et par analogie chez le lapin) une activité modérée a été mise en évidence avec une dose journalière de 2,8 g de sitostanol pendant un an.
   Or dans notre étude, l'apport en sitostanol est de 500 mg par jour, et l'activité est mise en évidence après 20 jours de traitement.
- l'huile d'olive : en effet dans l'étude d'intervention diététique de Lyon ( Pr Serge RENAUD) dans laquelle ont été inclus 600 patients ayant fait dans les six semaines précédentes un infarctus du myocarde et randomisés en deux groupes ; le groupe traité recevait des commandements diététiques remplaçant le beurre et les graisses animales par de l'huile d'olive. Après un suivi moyen de 27 mois, il est apparu que le groupe traité présentait une mortalité cardio-vasculaire réduite de 70%, mais aucune différence des paramètres lipidiques; la consommation d'huile d'olive n'avait pas fait baisser les taux de cholestérol.
   Les résultats sont bien en relation avec le complexe organo-métallique selon l'invention.

## Revendications

1. Complexe organométallique susceptible d'être obtenu par réaction :
- d'un cation d'un métal (M) au degré d'oxydation au moins égal à 2 utilisable comme biocatalyseur dans le métabolisme vivant,
- du sitostérol, du sitostanol ou d'un mélange de sitostérol et de sitostanol, ou d'un extrait végétal contenant du sitostérol, du sitostanol ou un mélange de sitostérol et de sitostanol,
- du diglycéride répondant à la formule (I): 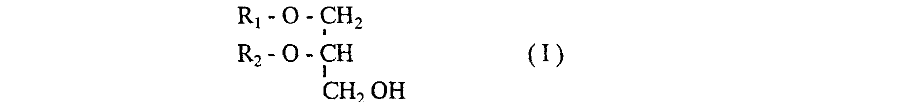
dans laquelle :
- R₁ est un reste acyle d'acide oleique ( C _{18 :1}),
- R2 est un reste acyle d'acide gras comprenant entre 2 et 18 atomes de carbone, linéaire ou ramifié, saturé ou insaturé.

2. Complexe organométallique selon la revendication 1, **caractérisé en ce que** le reste R₂ d'acide en C₂ à C₁₈ est un reste d'acide oleique.

3. Complexe selon la revendication 1, **caractérisé en ce que** le reste R₂ d'acide est un groupement acétyle.

4. Complexe selon l'une des revendications 1 à 3 , **caractérisé en ce que** le mélange de sitostérol et de sitostanol est obtenu à partir du soja.

5. Complexe selon l'une des revendications 1 à 3 , caracté risé en ce que le sitostanol est obtenu par hydrogénation du sitostérol.

6. Complexe selon l'une des revendications 1 à 5, **caractérisé en ce que** le diglycéride est obtenu par purification à partir de l'huile d'olive ou d'une huile riche en acide oleique.

7. Complexe selon l'une des revendications 1 à 6, **caractérisé en ce que** le métal appartient à la famille des métaux suivants : zinc, fer, cuivre, magnésium, vanadium, titane, chrome, manganèse, cobalt, nickel, gallium, germanium, antimoine, étain, indium, palladium, rhodium, ruthénium, technium, molybdène, niobium, zirconium, yttrium, tantale, tungstène, rhénium, osmium, iridium, platine, or, argent, thallium.

8. Complexe selon l'une des revendications 1 à 7 , **caractérisé en ce que** le métal est le vanadium au degré d'oxydation 3, 4 ou 5, de préférence 4.

9. Complexe selon l'une des revendications 1 à 8 , **caractérisé en ce que** le métal est obtenu par extraction à partir d'un végétal connu pour sa forte teneur en ce métal, ou encore par l'utilisation directe de ce végétal sous forme pulvérisée.

10. Procédé de préparation d'un complexe selon l'une des revendications 1 à 9 , **caractérisé en ce qu'**on mélange les constituants dudit complexe dans un solvant organique ou une huile.

11. Procédé selon la revendication 10, **caractérisé en ce que** l'on porte le dit mélange à une température comprise entre 30° et 40°C pendant une à plusieurs heures, ou à quelques expositions flash de quelques minutes à une température de l'ordre de 50° à 60°C.

12. Utilisation d'un complexe organométallique selon l'une des revendications 1 à 9, ou obtenu selon le procédé des revendications 10 ou 11, en tant qu'agent transporteur du cation M à activité biocatalytique.

13. Composition pharmaceutique **caractérisée en ce qu'**elle contient au moins un complexe tel que défini dans l'une des revendications 1 à 9, ou obtenu selon le procédé des revendications 10 ou 11, et un véhicule, excipient ou support pharmaceutiquement acceptable.

14. Composition selon la revendication 13, caractéri sée en ce qu'elle contient un complexe du vanadium.

15. Composition selon la revendication 13 ou 14, **caractérisée en ce que** ledit diglycéride entrant dans la composition dudit complexe est le 1-2-dioleoyl glycérol.

16. Composition selon la revendication 13 ou 14, **caractérisée en ce que** ledit diglycéride entrant dans la composition dudit complexe est le 1-oleoyl-2-acetyl glycérol.

17. Composition pharmaceutique et produit diététique **caractérisées en ce qu'**ils contiennent au moins un complexe tel que défini dans les revendications 1 à 5, dans laquelle l'acylglycérol est amené par l'huile d'olive qui constitue en même temps l'excipient, avec un procédé tel que décrit dans la revendication 11.

18. Utilisation d'un complexe selon l'une des revendications 1 à 9, ou obtenu selon l'une des revendications 10 ou 11, pour la préparation d'un médicament ou d'un produit diététique destiné à être utilisé comme agent régulateur ou stimulant des systèmes biocatalytiques, en particulier dans le traitement ou la prévention des déficits ou dysfonctionnements (génétiques ou acquis) des systèmes enzymatiques nécessitant la présence de métaux sous forme de cations comme catalyseur des réactions biochimiques notamment dans le traitement ou la prévention des maladies métaboliques, auto-immunes ou néoplasiques.

19. Utilisation selon la revendication 18 d'un complexe selon l'une des revendications 1 à 9, ou obtenu selon les procédés décrits dans les revendications 10 ou 11, dans lequel le métal est choisi dans le groupe constitué du vanadium, du niobium, du sélénium, du chrome, du zinc et du molybdène pour le traitement et/ou la prévention de l'hypercholestérolémie et/ou de l'hypertriglycéridémie, du diabète insulino-dépendant ou non insulino-dépendant, de l'insulino-résistance et de leurs complications cardio-vasculaires, en particulier l'hypertension artérielle, les coronaropathies obstructives , telles qu'infarctus du myocarde ou angor, les microangiopathies oculaires ou périphériques, les artériopathies oblitérantes périphériques, ainsi que de l'obésité de type androïde.

20. Utilisation selon la revendication 18 ou 19, **caractérisée en ce que** le métal est le vanadium, de préférence le vanadium au degré d'oxydation 3,4 ou 5, de préférence 4.

21. Utilisation selon la revendication 18 à 20, **caractérisé en ce que** le métal est amené par une plante possédant une teneur élevée dans ce métal, sous forme d'extrait ou de poudre de cette plante.

22. Utilisation selon l'une des revendications 18 à 21 , **caractérisée en ce que** le diglycéride est le 1,2-dileoyl glycérol ou le 1-oleoyl-2acétyl glycérol.

23. Utilisation selon l'une des revendications 18 à 22, **caractérisée en ce que** le stérol est du sitostérol, du sitostanol ou d'un mélange de sitostérol et de sitostanol.

## Patentansprüche

1. Metallorganischer Komplex, der erhältlich ist durch Umsetzung:
- eines Kations eines Metalls (M) in einer Oxidationsstufe von mindestens 2, das als Biokatalysator im in vivo-Stoffwechsel verwendet werden kann, mit
- Sitosterin, Sitostanol oder einer Mischung von Sitosterin und Sitostanol oder einem Pflanzenextrakt, der Sitosterin, Sitostanol oder eine Mischung von Sitosterin und Sitostanol enthält, und
- einem Diglycerid der Formel (I) 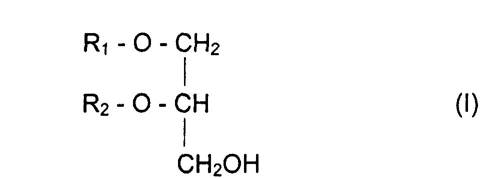
worin bedeuten:
R₁ einen Acylrest der (C_{18:1})-Ölsäure,
R₂ einen Rest einer linearen oder verzweigten, gesättigten oder ungesättigten Fettsäure mit 2 bis 18 Kohlenstoffatomen.

2. Metallorganischer Komplex nach Anspruch 1, **dadurch gekennzeichnet, dass** der C₂-C₁₈-Säurerest R₂ ein Ölsäurerest ist.

3. Komplex nach Anspruch 1, **dadurch gekennzeichnet, dass** der Säurerest R₂ eine Acetylgruppe ist.

4. Komplex nach einem der Ansprüche 1 bis 3, **dadurch gekennzeichnet, dass** die Mischung von Sitosterin und Sitostanol aus Soja hergestellt ist.

5. Komplex nach einem der Ansprüche 1 bis 3, **dadurch gekennzeichnet, dass** das Sitostanol durch Hydrierung von Sitosterin hergestellt worden ist.

6. Komplex nach einem der Ansprüche 1 bis 5, **dadurch gekennzeichnet, dass** das Diglycerid durch Reinigung aus Olivenöl oder einem an Ölsäure reichen Öl hergestellt worden ist.

7. Komplex nach einem der Ansprüche 1 bis 6, **dadurch gekennzeichnet, dass** das Metall zur Familie der folgenden Metalle gehört: Zink, Eisen, Kupfer, Magnesium, Vanadin, Titan, Chrom, Mangan, Kobalt, Nickel, Gallium, Germanium, Antimon, Zinn, Indium, Palladium, Rhodium, Ruthenium, Technetium, Molybdän, Niob, Zirkonium, Yttrium, Tantal, Wolfram, Rhenium, Osmium, Iridium, Platin, Silber und Thallium.

8. Verbindung nach einem der Ansprüche 1 bis 7, **dadurch gekennzeichnet, dass** das Metall Vanadin in der Oxidationsstufe 3, 4 oder 5, vorzugsweise 4, ist.

9. Komplex nach einem der Ansprüche 1 bis 8, **dadurch gekennzeichnet, dass** das Metall durch Extraktion aus einer Pflanze, die für ihren hohen Gehalt an diesem Metall bekannt ist, oder durch direkte Verwendung dieser Pflanze in pulverisierter Form erhalten wurde.

10. Verfahren zur Herstellung eines Komplexes nach einem der Ansprüche 1 bis 9, **dadurch gekennzeichnet, dass** man die Bestandteile des genannten Komplexes in einem organischen Lösungsmittel oder in einem Öl miteinander mischt.

11. Verfahren nach Anspruch 10, **dadurch gekennzeichnet, dass** man die genannte Mischung eine bis mehrere Stunden lang auf eine Temperatur zwischen 30 und 40°C bringt oder bei einer Temperatur in der Größenordnung von 50 bis 60°C Flashes von einigen Minuten aussetzt.

12. Verwendung eines metallorganischen Komplexes nach einem der Ansprüche 1 bis 9 oder eines solchen, der nach dem Verfahren nach Anspruch 10 oder 11 hergestellt worden ist, als Transportagens für das Kation M mit biokatalytischer Aktivität.

13. Pharmazeutische Zusammensetzung, **dadurch gekennzeichnet, dass** sie mindestens einen Komplex, wie er in einem der Ansprüche 1 bis 9 definiert ist, oder bei dem Verfahren der Ansprüche 10 oder 11 erhalten wird, und ein Vehiculum, einen Exzipienten oder einen Träger, der (das) pharmazeutisch akzeptabel ist, enthält.

14. Zusammensetzung nach Anspruch 13, **dadurch gekennzeichnet, dass** sie einen Vanadin-Komplex enthält.

15. Zusammensetzung nach Anspruch 13 oder 14, **dadurch gekennzeichnet, dass** es sich bei dem genannten Diglycerid, das in die Zusammensetzung des genannten Komplexes eintritt, um 1,2-Dioleoyl-glycerin handelt.

16. Zusammensetzung nach Anspruch 13 oder 14, **dadurch gekennzeichnet, dass** es sich bei dem genannten Diglycerid, das in die Zusammensetzung des genannten Komplexes eintritt, um 1-Oleoyl-2-acetyl-glycerin handelt.

17. Pharmazeutische Zusammensetzung und diätetisches Produkt, **dadurch gekennzeichnet, dass** sie mindestens einen Komplex enthalten, wie er in den Ansprüchen 1 bis 5 definiert ist, in den das Acylglycerin mittels Olivenöl, das gleichzeitig den Exzipienten darstellt, nach einem Verfahren, wie es in Anspruch 11 beschrieben ist, eingeführt worden ist.

18. Verwendung eines Komplexes nach einem der Ansprüche 1 bis 9 oder eines solchen, der nach einem der Ansprüche 10 und 11 hergestellt worden ist, zur Herstellung eines Arzneimittels oder eines diätetischen Produkts, das bestimmt ist für die Verwendung als regulierendes oder stimulierendes Agens der biokatalytischen Systeme, insbesondere für die Behandlung oder Prävention von Defiziten oder Dysfunktionen (genetischen oder erworbenen) von enzymatischen Systemen, welche die Anwesenheit von Metallen in Form von Kationen als Katalysator für biochemische Reaktionen erfordern, insbesondere für die Behandlung oder Prävention von Stoffwechsel-, Autoimmun- oder Neoplasie-Erkrankungen.

19. Verwendung nach Anspruch 18 eines Komplexes nach einem der Ansprüche 1 bis 9 oder eines solchen, der nach den in den Ansprüchen 10 oder 11 beschriebenen Verfahren hergestellt worden ist, in dem das Metall ausgewählt wird aus der Gruppe, die besteht aus Vanadin, Niob, Selen, Chrom, Zink und Molybdän, für die Behandlung und/oder Prävention der Hypercholesterinämie und/oder der Hypertriglyceridämie, des insulinabhängigen oder von Insulin nicht abhängigen Diabetes, der Insulinresistenz und ihrer cardiovasculären Komplikationen, insbesondere der arteriellen Hypertension, der obstruktiven Coronarerkrankungen, wie Myocard-Infakt oder Angor, der okulären oder peripheren Mikroangiopathien, der peripheren obliterierenden Arteriopathien sowie der Obesitas vom Android-Typ.

20. Verwendung nach Anspruch 18 oder 19, **dadurch gekennzeichnet, dass** es sich bei dem Metall um Vanadin, vorzugsweise um Vanadium in der Oxidationsstufe 3, 4 oder 5, vorzugsweise 4, handelt.

21. Verwendung nach den Ansprüchen 18 bis 20, **dadurch gekennzeichnet, dass** das Metall durch eine Pflanze, die einen erhöhten Gehalt an diesem Metall aufweist, in Form eines Extrakts oder eines Pulvers dieser Pflanze eingeführt worden ist.

22. Verwendung nach einem der Ansprüche 18 bis 21, **dadurch gekennzeichnet, dass** es sich bei dem Diglycerid um 1,2-Dioleoyl-glycerin oder 1-Oleoyl-2-acetyl-glycerin handelt.

23. Verwendung nach einem der Ansprüche 18 bis 22, **dadurch gekennzeichnet, dass** es sich bei dem Sterin um Sitosterin, Sitostanol oder eine Mischung von Sitosterin und Sitostanol handelt.

## Claims

1. Organometallic complex obtainable by reaction :
- of a cation of a metal (M) in an oxidation state at least equal to 2 usable as a biocatalyst in living metabolism,
- of sitosterol, of sitostanol, or of a mixture of sitosterol and sitostanol, or of a plant extract containing sitosterol, sitostanol or a mixture of sitosterol and sitostanol,
- of the diglyceride of formula (I): 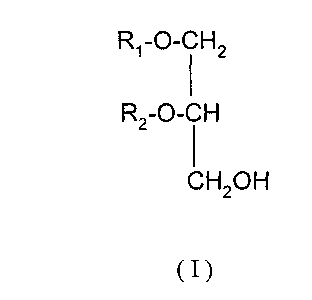 in which :
- R₁ is an acyl residue of oleic acid ( C₁₈ :1),
- R2 in an acyl residue of a linear or branched, saturated or unsaturated fatty acid having between 2 and 18 carbon atoms.

2. Organometallic complex according to claim 1, **characterised in that** the C₂ to C₁₈ acid residue R₂ is an oleic acid residue.

3. Complex according to claim 1, **characterised in that** the R₂ acid residue is an acetyl group.

4. Complex according to one of claims 1 to 3 , **characterised in that** the mixture of sitosterol and sitostanol is obtained from soya.

5. Complex according to one of claims 1 to 3 , **characterised in that** sitostanol is obtained by hydrogenation of sitosterol.

6. Complex according to one of claims 1 to 5, **characterised in that** the diglyceride is obtained by purification of olive oil or an oil rich in oleic acid.

7. Complex according to one of claims 1 to 6, **characterised in that** the metal belongs to following family of metals : zinc, iron, copper, magnesium, vanadium, titanium, chromium, manganese, cobalt, nickel, gallium, germanium, antimony, tin, indium, palladium, rhodium, ruthenium, technium, molybdenum, niobium, zirconium, yttrium, tantalum, tungsten, rhenium, osmium, iridium, platinum, gold, silver, thallium.

8. Complex according to one of claims 1 to 7 , **characterised in that** the metal is vanadium in an oxidation state of 3, 4 or 5, preferably 4.

9. Complex according to one of claims 1 to 8 , **characterised in that** the metal is obtained by extraction from a plant known for its high content of this metal, or even by the direct use of this plant in a pulverised form.

10. Method of preparation of a complex according to one of claims 1 to 9, **characterised in that** the constituents of said complex are mixed in an organic solvent or an oil.

11. Method according to claim 10, **characterised in that** said mixture is heated at a temperature between 30° and 40°C for one to several hours, or heated with a few flash exposures for a few minutes at a temperature of the order of 50° to 60°C.

12. Use of an organometallic complex according to one of claims 1 to 9, or obtained according to the method of claims 10 or 11, as M cation transporting agent with biocatalytic activity.

13. Pharmaceutical composition **characterised in that** it contains at least one complex as defined in one of claims 1 to 9, or obtained according to the method of claims 10 or 11, and a pharmaceutically acceptable vehicle, excipient or carrier.

14. Composition according to claim 13, **characterised in that** it contains a vanadium complex.

15. Composition according to claim 13 or 14, **characterised in that** said diglyceride entering in the composition of said complex is 1-2-dioleoyl glycerol.

16. Composition according to claim 13 or 14, **characterised in that** said diglyceride entering in the composition of said complex is 1-oleoyl-2-acetyl glycerol.

17. Pharmaceutical composition and dietary product **characterised in that** they contain at least one complex as defined in claims 1 to 5, in which the acylglycerol is provided by olive oil which constitutes at the same time the excipient, with a method as described in claim 11.

18. Use of a complex according to one of claims 1 to 9, or obtained according to one of claims 10 or 11, for the preparation of a medicament intended for use as regulating or stimulating agent of biocatalytic systems, particularly in the treatment or the prevention of deficiencies or dysfunctions (genetic or acquired) of enzymatic systems necessitating the presence of metals in the form of cations as catalyst of biochemical reactions especially in the treatment or the prevention of metabolic, auto-immune or neoplastic illnesses.

19. Use according to claim 18 of a complex according to one of claims 1 to 9, or obtained according to the methods described in claims 10 or 11, in which the metal is selected from the group consisting of vanadium, niobium, selenium, chromium, zinc and molybdenum for the treatment and/or the prevention of hypercholesterolaemia and/or of hypertriglyceridaemia, of insulin-dependent or non insulin-dependent diabetes, of insulin resistance and of their cardiovascular complications, particularly arterial hypertension, of obstructive coronaropathies, such as myocardial infarction or angor, of ocular or peripheral microangiopathies, of peripheral obliterating arteriopathies, as well as of android-type obesity.

20. Use according to claim 18 or 19, **characterised in that** the metal is vanadium, preferably vanadium in an oxidation state of 3,4 or 5, preferably 4.

21. Use according to one of claims 18 to 20 **characterized in that** the metal is brought by a plant having a high content of said metal, in the form of an extract or a powder of said plant.

22. Use according to one of claims 18 to 21 , **characterised in that** the diglyceride is 1,2-dileoyl glycerol or 1-oleoyl-2acetyl glycerol.

23. Use according to one of claims 18 to 22, **characterised in that** the sterol is sitosterol, sitostanol or a mixture of sitosterol and sitostanol.
